# EUROPEAN PATENT SPECIFICATION

(11) **EP 3 727 298 B1**
(45) Date of publication and mention of the grant of the patent: **07.06.2023**
(21) Application number: 18815160.9
(22) Date of filing: 04.12.2018
(51) Int. Cl.: A61K 8/29, A61Q 1/02, A61K 8/81, A61K 8/89, A61K 8/90, A61K 8/19, A61K 8/04

(54) **COLOURED AQUEOUS PARTICLE DISPERSION**
GEFÄRBTE WÄSSRIGE PARTIKEL-DISPERSION
DISPERSION AQUEUSE COLOREE

(30) Priority: 20.12.2017 FR 1762598
(43) Date of publication of application: 28.10.2020
(73) Proprietor: L'OREAL, 75008 Paris (FR)
(72) Inventor: EL ACHKAR, Micheline, 94152 CHEVILLY LA RUE (FR); MARCAIS-HELIE, Caroline, 94152 Chevilly Larue (FR)
(74) Representative: L'Oreal
(86) International application number: PCT/EP2018/083478
(87) International publication number: WO 2019/120985

(56) References cited:
- DATABASE GNPD [Online] MINTEL; 3 July 2017 (2017-07-03), L'Oréal: "Fixing Mist Make-Up Finishing Spray", XP002781178, Database accession no. 4974293
- DATABASE GNPD [Online] MINTEL; 30 September 2016 (2016-09-30), anonymous: "Water Blend Face & Body Foundation", XP055792320, Database accession no. 4328353
- DATABASE GNPD [Online] MINTEL; 11 May 2007 (2007-05-11), anonymous: "Mousse Mascara", XP055792246, Database accession no. 704667

## Description

The present invention is directed towards proposing, for the field of caring for and/or making up keratin materials, especially the skin, a novel coloured aqueous particle dispersion that is most particularly advantageous with regard to its technical performance and the sensations it affords the user on application to said keratin materials and in particular to the skin.

Cosmetic compositions, for example foundations, are commonly used to give the skin an aesthetic colour, but also to enhance the beauty of irregular skin, by making it possible to hide marks and dyschromias, to reduce the visibility of relief imperfections such as pores and wrinkles, and to conceal spots, acne marks and scars. In this regard, the coverage and the matt effect or "soft focus" effect are the main properties desired.

Emulsions are generally appealing to consumers, in particular in the context of foundations, since they are easy to apply. For such compositions, it is sought to provide colour to enhance the beauty of the skin. Substantial coverage and a matt effect are also sought to hide skin defects and to make the complexion uniform. A high proportion of pigments is generally used for this purpose. It is also important for the pigments present in these compositions to have good dispersibility with a view to obtaining a stable and uniform composition. In these emulsions, consumers are also looking for products which, after application, afford a light texture with no thickness or heaviness effect and which do not transfer onto substrates in contact with the keratin materials such as the skin, for instance clothing. However, the emulsions generally used in makeup have a tendency to have poor performance as regards freshness, and to produce a greasy effect due to the large presence of oils in these emulsions. Furthermore, the light texture effect and/or the transfer-resistance effect are not always fully satisfactory.

Mintel Citation "Water Blend Face & Body Foundation" published on 2016-09-30 is considered to be the closest prior art document, because it discloses a foundation comprising iron oxide and titanium dioxide pigments, Acrylates/C10-30 Alkyl Acrylate Crosspolymer as a thickening agent.

There remains a need to find novel aqueous presentation forms based on pigments that can be used especially as foundations which have good dispersibility of the pigments so as to obtain a homogeneous composition, and which give good sensory properties in terms of freshness, a non-greasy effect, a light texture effect and a transfer-resistance effect, while at the same time having good makeup properties such as good colour coverage, a good matt effect and good persistence of these effects

The Applicant has discovered, surprisingly, that this objective can be achieved with a composition in the form of an aqueous particle dispersion, especially comprising a physiologically acceptable medium, especially for coating keratin materials, more particularly for making up and/or caring for keratin materials, such as the skin, containing:
a) an aqueous phase; and
b) at least one matt-effect filler **chosen from boron nitride, polymethylsilsesquioxane powders, and mixtures thereof;**
**c) solid particles formed from a film-forming polymer suspended in the aqueous phase which are an aqueous dispersion of aminomethylpropanol salt of a copolymer of allyl methacrylate and of one or more monomers chosen from acrylic acid, methacrylic acid or an ester thereof of INCI name: AMP-acrylates/allyl methacrylate copolymer; and**
d) at least one ionic polymeric dispersant **chosen from homopolymers of the sodium salt of methacrylic acid;**
e) at least one thickener **chosen from:**
   - **copolymers of at least two monomers chosen from acrylic acid, methacrylic acid or an ester;**
   - **polyacrylic acid/C₁₀-C₃₀ alkyl acrylate crosslinked copolymers;**
   - **an acrylamidomethylpropanesulfonic acid polymer partially neutralized with ammonia and crosslinked, and mixtures thereof, and**
f) at least one particulate dyestuff **chosen from titanium dioxides, iron oxides, and mixtures thereof.**

This discovery forms the basis of the invention.

Thus, according to one of its aspects, the present invention relates to a composition in the form of an aqueous dispersion of solid particles, especially comprising a physiologically acceptable medium, especially for coating keratin materials, more particularly for making up and/or caring for keratin materials, such as the skin, containing:
a) an aqueous phase; and
b) at least one matt-effect filler **chosen from boron nitride, polymethylsilsesquioxane powders, and mixtures thereof ;**
**c) solid particles formed from a film-forming polymer suspended in the aqueous phase which are an aqueous dispersion of aminomethylpropanol salt of a copolymer of allyl methacrylate and of one or more monomers chosen from acrylic acid, methacrylic acid or an ester thereof of INCI name: AMP-acrylates/allyl methacrylate copolymer; and**
d) at least one ionic polymeric dispersant **chosen from homopolymers of the sodium salt of methacrylic acid ;**
e) at least one thickener **chosen from:**
   - **copolymers of at least two monomers chosen from acrylic acid, methacrylic acid or an ester ;**
   - **polyacrylic acid/C₁₀-C₃₀ alkyl acrylate crosslinked copolymers ;**
   - **an acrylamidomethylpropanesulfonic acid polymer partially neutralized with ammonia and crosslinked, and mixtures thereof, and**
f) at least one particulate dyestuff **chosen from titanium dioxides, iron oxides, and mixtures thereof.**

The invention also relates to a process for coating keratin materials, more particularly for making up and/or caring for keratin materials, such as the skin, characterized in that it comprises the application to the keratin materials of a composition as defined previously.

### DEFINITIONS

In the context of the present invention, the term "keratin material" especially means the skin (of the body, face, or around the eyes).

The term "physiologically acceptable" means compatible with the skin and/or its integuments, which has a pleasant colour, odour and feel, and which does not cause any unacceptable discomfort (stinging or tautness) liable to discourage the consumer from using this composition.

The term "aqueous particle dispersion" means any composition comprising an aqueous phase in which are dispersed particles that are insoluble in said aqueous phase; said composition not comprising any other phase that is immiscible in the aqueous phase of the composition, irrespective of the temperature at which the composition is manufactured.

The term "particle" denotes a solid material that is insoluble in the aqueous phase of the composition, irrespective of the temperature at which the composition is manufactured.

The term "fillers" should be understood as meaning colourless or white, mineral or synthetic particles of any shape, which are insoluble and dispersed in the aqueous phase of the composition, irrespective of the temperature at which the composition is manufactured.

### MATT-EFFECT OR "SOFT-FOCUS" FILLER

The term matt-effect or "soft-focus" filler means a filler which gives the complexion greater transparency and a hazy effect. The matt-effect power of the compositions containing same may be characterized by means of the following protocol:
According to the invention, the term "matt-effect power" especially means that the composition produces a negative sheen value measured using a polarimetric camera, after application of a composition according to the invention to said keratin material.

The matt-effect fillers used in the compositions according to the present invention may be in lamellar (or platelet), spherical (or globular) form, in the form of fibres or in any other intermediate form between these defined forms.

In the present patent application, the term "spherical particles" means particles in the shape or substantially in the shape of a sphere, which are insoluble in the medium of the composition according to the invention, even at the melting point of the medium (approximately 100°C).

The term "lamellar particles" means herein particles of parallelepipedal shape (rectangular or square surface), discoid shape (circular surface) or ellipsoid shape (oval surface), characterized by three dimensions: a length, a width and a height, which particles are insoluble in the medium of the composition according to the invention, even at the melting point of the medium (approximately 100°C).

The matt-effect filler(s) used in the compositions according to the present invention are preferably present in concentrations ranging from 0.1% to 5% by weight and more preferentially from 1% to 3% by weight relative to the total weight of the composition.

According to **the invention**, the filler(s) are chosen from boron nitride, and mixtures thereof.

By way of example, these matt-effect fillers may be chosen from: - polymethylsilsesquioxane powders such as those having the INCI name: Polymethylsilsesquioxane, such as the products sold under the name Tospearl 145A^{®} by the company GE Silicone;

### Boron nitride

There are several polymorphic forms of boron nitride: hexagonal form boron nitrides (denoted h-BN), rhombohedral form boron nitrides (denoted r-BN), amorphous form boron nitrides (denoted a-BN), turbostratic boron nitrides (denoted t-BN), cubic form boron nitrides (denoted c-BN) and wurtzite-type hexagonal form boron nitrides (denoted w-BN).

Preferentially, the boron nitride particles in accordance with the invention are chosen from turbostratic boron nitride particles, i.e. particles whose crystallization planes may be slightly offset relative to the theoretical position of crystallization. Turbostratic boron nitride is a precursor of hexagonal form born nitride (h-BN). It has the same type of characteristics and physical properties as exfoliated hexagonal boron nitride.

Preferentially, the boron nitride particles have an oxygen content ranging from 0.05% to 3% by weight, more preferentially from 0.1% to 2.5% by weight, relative to the total weight of the particle.

Preferentially, the boron nitride particles have a mean particle size ranging from 0.1 to 25 µm, preferably from 0.3 to 15 µm.

The particle size is measured according to a method of distribution by laser diffraction with an apparatus of the type Microtrac from Nikkiso or Mastersizer from Malvern, in particular by measuring the D[10], D[50] and D[90] values.

D[10] represents the maximum size that 10% by volume of the particles have.

D[50] represents the maximum size that 50% by volume of the particles have.

D[90] represents the maximum size that 90% by volume of the particles have.

The boron nitride particles can be modified with a surface-treatment agent making it possible to confer thereon amphiphilic properties and to promote the dispersibility thereof in the compositions comprising an oily phase and/or an aqueous phase.

Treatment agents that may be chosen include dimethylpolysiloxanes (dimethicone), linear siloxane polymers end-blocked with trimethoxysiloxy groups, polymethylhydrogenosiloxanes which are linear polysiloxanes called methicones, and polyoxyalkylenated polyalkylethersiloxanes such as the polymer PEG-8 methyl ether dimethicone.

The boron nitride particles in accordance with the invention may be chosen more particularly from the following commercial products:
UHP-1010^{®} from Carborundum,
PUHP 1030L^{®} from the company Saint Gobain Ceramics,
Boron Nitride Powder TRES BN PUHP 3002^{®} from the company Saint Gobain Ceramics,
TRES BN PUHP 30005^{®} from the company Saint Gobain Ceramics,
Leau3002^{®} (INCI name: Boron Nitride (and) PEG-8 Methyl Ether Dimethicone) from the company Saint Gobain,
Ronaflair Boroneige SF-3 117774^{®} from the company Merck,
Ronaflair Boroneige SQ-6^{®} from MERCK,
Softouch Boron Nitride CC6059^{®} from the company Momentive,
Softtouch Boron Nitride CC 6097^{®} from the company Momentive,
Softtouch CCS102J^{®} from the company Momentive.

### FILM-FORMING POLYMER PARTICLES

The composition according to the invention comprises solid particles formed from one or more film-forming polymers suspended in the aqueous phase of the composition.

Said solid film-forming polymer particles either may be used per se and are in suspension in the aqueous phase of the composition or may be used in the form of particles in aqueous dispersion (latex or pseudolatex).

In the present patent application, the term "film-forming polymer" means a polymer that is capable of forming, by itself or in the presence of an auxiliary film-forming agent, a continuous film on a support, at a temperature ranging from 20°C to 150°C.

Such a film-forming polymer present in the form of particles in aqueous dispersion is generally known as a (pseudo)latex, i.e. a latex or pseudolatex. Techniques for preparing these dispersions are well known to those skilled in the art.

The composition according to the invention may comprise one or more types of particle, these particles possibly varying as regards their size, their structure and/or their chemical nature.

The film-forming polymer(s) are preferentially present in a solids content ranging from 0.1% to 5% by weight, more preferentially ranging from 0.1% to 3% by weight, even more preferentially ranging from 0.1% to 2% by weight and more particularly from 0.1% to 1.5% by weight relative to the total weight of the composition.

In the present invention, the term "aqueous" refers to a liquid medium based on water and/or hydrophilic solvents. This aqueous liquid medium may be constituted essentially of water. It may also comprise a mixture of water and of water-miscible organic solvent(s) (miscibility with water of greater than 50% by weight at 25°C), for instance lower monoalcohols containing from 1 to 5 carbon atoms such as ethanol or isopropanol, glycols containing from 2 to 8 carbon atoms such as propylene glycol, ethylene glycol, 1,3-butylene glycol and dipropylene glycol, C₃-C₄ ketones and C₂-C₄ aldehydes.

Among the film-forming polymers that may be used in the composition of the present

According to the invention, use will be made of an aqueous dispersion of aminomethylpropanol salt of a copolymer of allyl methacrylate and of one or more monomers chosen from acrylic acid, methacrylic acid or an ester thereof of INCI name: AMP acrylates/allyl methacrylate copolymer, for instance the commercial product Fixate G-100L PR Polymer@ in the form of an aqueous dispersion at 25-28% by weight by the company Lubrizol Advanced Materials, Inc.).

### IONIC POLYMERIC DISPERSANT

The ionic polymeric dispersants are **chosen from (meth)acrylic acid homopolymers and salts thereof.**

The term "salts" means the salts of an alkali metal such as Na, Li or K of said acids, the salts of monoethanolamine, diethanolamine or triethanolamine or basic amino acids such as lysine or arginine of said acids, and mixtures thereof.

Use will be made more particularly of a polymer of the sodium salt of methacrylic acid of INCI name: Sodium polymethacrylate, especially as an aqueous 25% solution sold under the reference Darvan^{®} 7-N by the company R.T. Vanderbilt Company, Inc.

A composition according to the invention preferably includes a total content of ionic polymeric dispersant(s) of greater than or equal to 0.05% by weight and less than or equal to 3% by weight, relative to the total weight of the composition, in particular ranging from 0.1% to 2% by weight, relative to the total weight of the composition, more particularly ranging from 0.1% to 1.5% by weight and even more particularly ranging from 0.1% to 1% by weight, relative to the total weight of the composition.

### AQUEOUS PHASE

The composition according to the invention comprises an aqueous phase.

The term "aqueous phase" means a phase comprising water and also all the water-soluble or water-miscible solvents and ingredients.

The aqueous phase may contain a demineralized water or alternatively a floral water such as cornflower water and/or a mineral water such as Vittel water, Lucas water or La Roche Posay water and/or a spring water.

According to a preferential embodiment, the aqueous phase is present in a content ranging from 70% to 95% by weight relative to the total composition, preferably in a content ranging from 80% to 90% by weight relative to the total weight of the composition.

According to a preferential embodiment, water is present in a content ranging from 40% to 80% by weight relative to the total composition, preferably in a content ranging from 60% to 70% by weight relative to the total weight of the composition.

### THICKENER

For the purposes of the present invention, the term "thickener" means any organic or inorganic compound that is capable of increasing the viscosity of the aqueous composition according to the invention.

The thickeners in accordance with the invention are preferably water-soluble.

For the purposes of the invention, the term "water-soluble thickener" means any thickener that can be fully dissolved in molecular form or that is miscible in a liquid aqueous phase or that can be dissolved in colloidal form (for example in micellar form) in a liquid aqueous phase.

Use will more particularly be made of a thickener chosen from:
- copolymers of at least two monomers chosen from acrylic acid, methacrylic acid or an ester thereof of INCI name Acrylates copolymer, in particular the product sold under the name Carbopol Aqua SF1^{®} by the company Lubrizol Advanced Materials, Inc.;
- polyacrylic acid/C10-C30 alkyl acrylate crosslinked copolymers of INCI name: Acrylates/C10-C30 alkyl acrylate crosspolymer, such as the products sold under the trade names Pemulen TR1 and Pemulen TR2^{®} or Carbopol 1382^{®}, Carbopol ETD 2020 Polymer@, Carbopol 1342 Polymer^{®}, Carbopol SC 200^{®}, Carbopol SC 500 Polymer@, Carbopol Ultrez 20 Polymer@, Carbopol Ultrez 21 Polymer@ from the company Lubrizol Advanced Materials, Inc.;
- an acrylamidomethylpropanesulfonic acid polymer partially neutralized with ammonia and crosslinked, of INCI name: Ammonium polyacryloyldimethyl taurate sold under the trade name Hostacerin AMPS^{®} by the company Clariant,
- and mixtures thereof.

The thickener(s) may preferably be used in a proportion of from 0.05% to 3% by weight of solids relative to the total weight of the aqueous phase, especially from 0.1 % to 2% by weight relative to the total weight of the composition.

### PARTICULATE DYESTUFF

The particulate dyestuff is intended in particular to give a coloured appearance or a nacreous effect to compositions that are useful for making up the skin.

The particulate dyestuff is preferably chosen from pigments and nacres, and mixtures thereof.

This dyestuff is preferably present in contents ranging from 5% to 40% by weight and more particularly from 10% to 30% by weight relative to the total weight of the composition. According to a preferred mode of the invention, the dyestuff is present in the composition in a total content ranging from 15% to 25% by weight relative to the total weight of the composition.

### Pigments

The term "pigments" should be understood as meaning white or coloured, mineral or organic particles of any shape, which are insoluble in the medium of the composition, and which are intended to colour the composition.

The pigments may be white or coloured, and mineral and/or organic.

The term "mineral pigment" means any pigment that satisfies the definition in Ullmann's encyclopaedia in the chapter on inorganic pigments. Among the mineral pigments that are useful in the present invention, mention may be made of iron oxide (black, yellow or red), **and** titanium dioxide.,

According to a particular mode, the pigment(s) used may be surface-treated and may especially be coated with at least one hydrophilic compound.

For the purposes of the invention, the "coating" of a pigment according to the invention generally denotes the total or partial surface treatment of the pigment with a surface agent absorbed, adsorbed or grafted onto said pigment.

The surface-treated pigments may be prepared according to surface treatment techniques of chemical, electronic, mechanochemical or mechanical nature that are well known to a person skilled in the art. Commercial products may also be used.

The surface agent may be absorbed, adsorbed or grafted onto the pigments by evaporation of solvent, chemical reaction and creation of a covalent bond.

According to one variant, the surface treatment is constituted of a coating of the pigments.

The coating may represent from 0.1% to 20% by weight and in particular from 0.5% to 10% by weight relative to the total weight of the coated pigment.

The coating may be realized, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be realized, for example, by chemical reaction of a surface agent with the surface of the solid pigment particles and creation of a covalent bond between the surface agent and the particles. This method is notably described in the patent US 4,578,266.

The chemical surface treatment may consist in diluting the surface agent in a volatile solvent, dispersing the pigments in this mixture and then slowly evaporating off the volatile solvent, so that the surface agent is deposited at the surface of the pigments.

The surface treatment agent may be hydrophilic, such as those described, for example, in Cosmetics & Toiletries, February 1990, Vol. 105, pages 53-64. Mention may be made, for example, of amino acids, C1-C5 alkanolamines, silicon oxides (silica), sodium hexametaphosphate or glycerol or mixtures thereof, PEG-12 dimethicone, sodium glycerophosphate, PEG-7 glyceryl cocoate + methylsilanol tri-PEG-8 glyceryl cocoate + polyquaternium-7, chitosan, methoxy PEG-10 propyltrimethoxysilane, PEG/PPG-18/18 dimethicone, microcrystalline cellulose, and polyethylene glycol alkoxysilanes.

A composition according to the invention may comprise a content of pigments ranging from 0% to 30% by weight relative to the total weight of the composition, preferably ranging from 0% to 20% by weight and preferentially ranging from 1% to 15% by weight, relative to the total weight of the composition. According to a preferred mode of the present invention, the pigments are present in the composition in a content ranging from 1% to 10% by weight relative to the total weight of the composition.

According **to the invention,** the particulate dyestuff will be chosen from titanium dioxide particles, iron oxide particles, and mixtures thereof.

According to a particularly preferred form of the invention, the composition in the form of an aqueous particle dispersion especially comprising a physiologically acceptable medium, especially for coating keratin materials, more particularly for making up and/or caring for keratin materials, such as the skin, contains:
a) an aqueous phase; and
b) at least one matt-effect filler chosen from boron nitride, polymethylsilsesquioxane powders, and mixtures thereof;
c) as solid particles formed from a film-forming polymer suspended in the aqueous phase, at least one aqueous dispersion of aminomethylpropanol salt of a copolymer of allyl methacrylate and of one or more monomers chosen from acrylic acid, methacrylic acid or an ester thereof of INCI name: AMP-acrylates/allyl methacrylate copolymer; and
d) at least one ionic polymeric dispersant chosen from homopolymers of the sodium salt of methacrylic acid;
e) at least one thickener chosen from:
   - copolymers of at least two monomers chosen from acrylic acid, methacrylic acid or an ester thereof;
   - polyacrylic acid/C₁₀-C₃₀ alkyl acrylate crosslinked copolymers;
   - an acrylamidomethylpropanesulfonic acid polymer partially neutralized with ammonia and crosslinked; and mixtures thereof, and
f) at least one particulate dyestuff chosen from titanium dioxides, iron oxides, and mixtures thereof.

### ADDITIVES

The compositions according to the invention may also comprise additional cosmetic ingredients conventionally used in aqueous cosmetic compositions especially for making up and/or caring for the skin. Examples that may especially be mentioned include additives chosen from polyols, monoalcohols, water-soluble dyestuffs, preserving agents, antioxidants, chelating agents, sequestrants, antifoams, fragrances, sunscreens, bactericidal agents, water-soluble active agents, and mixtures thereof.

Needless to say, a person skilled in the art will take care to select the optional additional ingredients and/or the amount thereof such that the advantageous properties of the composition according to the invention are not, or are not substantially, adversely affected by the envisaged addition.

More particularly, the compositions according to the invention contain at least one additive chosen from polyols, monoalcohols, and mixtures thereof.

### POLYOL

The composition may also comprise a polyol that is miscible with water at room temperature (25°C) chosen especially from polyols containing from 2 to 20 carbon atoms, preferably containing from 2 to 10 carbon atoms and preferentially containing from 2 to 8 carbon atoms, such as glycerol, propylene glycol, 1,3-butylene glycol, pentylene glycol, hexylene glycol, caprylyl glycol, dipropylene glycol or diethylene glycol; glycol ethers (especially containing from 3 to 16 carbon atoms) such as mono-, di- or tripropylene glycol (C₁-C₄)alkyl ethers, mono-, di- or triethylene glycol (C₁-C₄)alkyl ethers; polyethylene glycols; and mixtures thereof.

Use will more preferentially be made of propylene glycol.

According to a preferential mode of the invention, the polyol(s) are preferably present in a content ranging from 1% to 20% by weight relative to the total weight of the composition, and more particularly ranging from 5% to 15% by weight relative to the total weight of the composition.

### MONOALCOHOL

In addition, the composition according to the invention may comprise a monoalcohol containing from 2 to 5 carbon atoms, such as ethanol or isopropanol.

Ethanol will be used more preferentially.

According to a preferential mode of the invention, the monoalcohol is present in a content ranging from 1% to 10% by weight relative to the total weight of the composition, and preferably in a content ranging from 3% to 7% by weight relative to the total weight of the composition.

### ADDITIONAL DYESTUFFS

According to a particularly preferred mode of the invention, the compositions also contain at least one water-soluble dyestuff.

For the purposes of the invention, the term "water-soluble dyestuff' means any natural or synthetic, generally organic compound, which is soluble in an aqueous phase or water-miscible solvents and which is capable of colouring.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophyllin, methylene blue, anthocyanins (enocianin, black carrot, hibiscus and elder), caramel and riboflavin.

### COSMETIC APPLICATIONS

According to one embodiment, a composition of the invention may advantageously be in the form of a composition for caring for the skin of the body or the face, in particular the face.

According to another embodiment, a composition of the invention may advantageously be in the form of a composition for making up the skin of the body or the face, in particular the face.

A composition of the invention may advantageously be in the form of a foundation.

According to another mode of this embodiment, a composition of the invention may advantageously be in the form of an eyeshadow or a face powder.

Such compositions are especially prepared according to the general knowledge of those skilled in the art.

### COSMETIC KIT

According to another aspect, the invention also relates to a cosmetic assembly comprising:
i) a container delimiting at least one compartment, said container being closed by a closing member, and
ii) a composition as described previously, placed inside said compartment.

The container may be in any suitable form. It may especially be in the form of a bottle, a tube, a jar, a case, a can, a sachet or a box.

The closing member may be in the form of a removable stopper, a lid, a cap, a tear-off strip or a capsule, especially of the type including a body attached to the container and a cover cap articulated on the body. It may also be in the form of a member for selectively closing the container, especially a pump, a valve or a flap valve.

The container may be combined with an applicator. The applicator may be in the form of a fine brush, as described, for example, in patent FR 2 722 380. The applicator may be in the form of a foam or elastomer pad, of a felt-tipped pen or of a spatula. The applicator may be free (powder puff or sponge) or securely fastened to a stem borne by the closing member, as described, for example, in patent US 5 492 426. The applicator may be solidly attached to the container, as described, for example, in patent FR 2 761 959.

The product may be contained directly in the container, or indirectly. By way of example, the product may be arranged on an impregnated support, especially in the form of a wipe or a pad, arranged (individually or in plurality) in a box or in a sachet. Such a support incorporating the product is described, for example, in patent application WO 01/03538.

The closing member may be coupled to the container by screwing. Alternatively, the coupling between the closing member and the container is done other than by screwing, especially via a bayonet mechanism, by click-fastening, clamping, welding, adhesive bonding or by magnetic attraction. The term "click-fastening" is understood to mean, in particular, any system that involves surmounting a rim or bead of material by elastic deformation of a portion, especially of the closing member, then by returning to the elastically unstressed position of said portion after the rim or bead has been surmounted.

The container may be at least partially made of thermoplastic material. Examples of thermoplastic materials that may be mentioned include polypropylene and polyethylene.

Throughout the description, including the claims, the term "including a" should be understood as being synonymous with "including at least one", unless otherwise specified.

The expressions "between ... and ...", and "ranging from ... to ..." should be understood as meaning limits included, unless otherwise specified.

The invention is illustrated in greater detail by the examples and figures presented below. Unless otherwise indicated, the amounts shown are expressed as weight percentages.

### EXAMPLE

### Example 1: Foundation in the form of an aqueous particle dispersion

| Phase | INCI name | Concentration in % by weight |
|---|---|---|
| A1 | Water | 25.00 |
| | Acrylates/C10-C30 Alkyl Acrylate Crosspolymer (Carbopol 1342 Polymer^{®}) | 0.07 |
| | Ammonium Polyacryloyldimethyl Taurate (Hostacerin AMPS^{®}) | 1.00 |
| | Triethanolamine | 0.13 |
| | Sodium polymethacrylate (Darvan-7N^{®}) | 0.95 |
| | Propylene glycol | 8.00 |
| | Synthetic fluorophlogopite | 1.43 |
| | Titanium dioxide (CI: 77891) | 8.90 |
| | Yellow iron oxides (CI: 77492) | 1.21 |
| | Red iron oxides (CI: 77491) | 0.26 |
| | Black iron oxides (CI: 77499 ) | 0.12 |
| A2 | Water | qs 100 |
| | AMP-Acrylates/allyl methacrylate copolymer (Fixate G-100L PR Polymer^{®}) as an aqueous dispersion at 25-28% by weight | 5.00 |
| | Phenoxyethanol | 0.64 |
| | Disodium EDTA | 0.5 |
| | Chlorphenesin | 0.25 |
| | Dimethicone (and) polysorbate 65 (and) simethicone | 0.15 |
| | Acrylates copolymer (Carbopol Aqua SF-1 Polymer^{®}) | 0.5 |
| B | Polymethylsilsesquioxane (Tospearl 145A^{®}) | 2.00 |
| | Boron nitride (Boron Nitride SHP 3^{®}) | 1.00 |
| C | Denat. alcohol | 5.00 |

### Procedure:

The composition was manufactured at room temperature (25°C) in the following manner.

Phase A1 was prepared by introducing the two thickening polymers into the water in a tank with stirring using a paddle mixer for 15 minutes at 4000 rpm, then the triethanolamine was added with stirring for 5 minutes at the same speed. The propylene glycol and the sodium polymethacrylate were successively added with stirring for 5 minutes at the same speed, then the pigments and the synthetic mica were added at the same speed. After one minute, the walls were scraped and turbomixing was performed for 20 minutes at the same speed. Phase A2 was prepared by introducing the various ingredients into the water, which was added to the tank containing phase A1. Phase B and phase C were then successively added to the resulting mixture with stirring for 20 minutes at the same speed.

An aqueous dispersion in which the pigments are uniformly dispersed was obtained.

### Tests of the matt effect and the persistence of the matt effect

### Measurement principle

The gloss of the skin is measured using the Samba FDT_Fr2 polarimetric camera, which is a black and white polarimetric imaging system, with which images are acquired in parallel (P) and crossed (C) polarized light. By analysing the image resulting from subtraction of the two images (P-C), the gloss is quantified, by measuring the average greyscale of the brightest 5% of pixels corresponding to the gloss areas.

### Test procedure

The test proceeds in the following manner:
The test is performed *in vivo* on 17 Caucasian individuals from 18 to 65 years old, with greasy, sparingly to moderately wrinkled skin, a pale flesh tone, and toned skin with no wrinkles or redness on the cheeks. Each volunteer receives two products for half-face testing. This test is performed in parallel groups and the attribution of the individuals to the treatment groups is randomized.

The participants arrive in an air-conditioned waiting room (22°C ± 2°C) 15 minutes before the start of the test. They remove their makeup and an image of one of their cheeks is taken with the polarimetric camera. This image allows measurement of the gloss at T0 before applying makeup.

Next, 100 mg of foundation are weighed out on a watch glass, and are applied with the bare fingers to the half-face on which the measurement at T0 was taken.

After a drying time of 15 minutes, an image of the made-up cheek is taken with the polarimetric camera. This image allows measurement of the gloss just after applying makeup (Timm).

The models then return to the air-conditioned room for 3 hours.

Finally, an image of the made-up cheek after the waiting time of 3 hours is taken with the polarimetric camera. This image allows measurement of the gloss after 3 hours of makeup (T3h).

### Expressing the results

The difference Δ**E** (Timm - T0) which measures the effect of the makeup on the skin is calculated. A negative value means that the makeup reduces the gloss of the skin and that it thus has a matt effect.

The difference Δ**E** (T3h - Timm) which measures the persistence of this effect is then calculated. The value obtained should be as low as possible, which means that the makeup effect does not change over time.

The results obtained are shown in the table below.

| **Matt effect of Example 1** | **Immediate matt effect ΔE (Timm-To)** | **Matt effect persistence ΔE (T3h-Timm)** |
|---|---|---|
| | -6.43 ± 0.95 | 1.25 ± 0.88 |

The composition according to the invention had a good matt effect and good persistence of this effect after 3 hours.

### Tests of colour and of matt effect persistence

### Measurement principle

The colouring of the skin is measured using a skin image acquisition device such as the Chromasphere^{®} machine as described in patent EP1288706 and according to the method indicated in said document.

### Test procedure

The test proceeds in the following manner:
The test is performed *in vivo* on 17 Caucasian individuals from 18 to 65 years old, with greasy, sparingly to moderately wrinkled skin, a pale flesh tone, and toned skin with no wrinkles or redness on the cheeks. Each volunteer receives two products for half-face testing. This test is performed in parallel groups and the attribution of the individuals to the treatment groups is randomized.

The participants arrive in an air-conditioned waiting room (22°C ± 2°C) 15 minutes before the start of the test. They remove their makeup and the colour of one of their cheeks at T0 before being made up is evaluated using the Chromasphere^{®} machine.

Next, 100 mg of foundation are weighed out on a watch glass, and are applied with the bare fingers to the half-face on which the measurement at T0 was taken.

After a drying time of 15 minutes, the immediate colouring of the cheek after application (Timm) is measured.

The models then return to the air-conditioned room for 3 hours.

The immediate colouring of the skin after application of the made-up cheek is measured after a waiting period of 3 hours using the Chromasphere^{®} machine.

This image allows measurement of the colouring of the made-up cheek after 3 hours of makeup (T3h).

### Expressing the results

The difference ΔE (Timm - T0) which measures the effect of the makeup on the skin is calculated.

The difference ΔE (T3h - Timm) which measures the persistence of this colour effect is then calculated. The value obtained should be as low as possible, which means that the colour effect does not change over time.

The results obtained are shown in the table below.

| **Colouring effect of Example 1** | **Immediate colouring effect ΔE (Timm-To)** | **Persistence of the colouring ΔE (T3h-Timm)** |
|---|---|---|
| | 3.60 ± 0.46 | 0.64 ± 0.11 |

The composition according to the invention had a good colouring effect and good persistence of this effect after 3 hours.

### Comparative sensory test of transfer-resistance effect

The transfer-resistance effect of the composition of Example 1 according to the invention in the form of an aqueous coloured particle dispersion was compared with that of two reference products on the market of water-based foundations in the form of a water-in-oil emulsion, namely:
1) the product **WATER BLEND FACE & BODY FOUNDATION^{®}** (i.e.: Mintel product No.4792105) the ingredient list of which is:
   WATER, CI 77891, HYDROGENATED POLYISOBUTENE, SORBITAN TRIOLEATE, PENTAERYTHRITYL TETRAISOSTEARATE, METHYLPROPANEDIOL, GLYCERIN, FRAGRANCE, MANGIFERA INDICA SEED BUTTER, BUTYLENE GLYCOL, PHENOXYETHANOL, GLYCERYL CAPRYLATE, TROMETHAMINE, CI 77492, ACRYLATES/C10-30 ALKYL ACRYLATE CROSSPOLYMER, 1,2-PENTANEDIOL, PANTHENOL, ALUMINUM DIMYRISTATE, CI 77491, CI 77499, BUTYLATED HYDROXYTOLUENE, TOCOPHEROL (VITAMIN E);
2) the product **LIQUID AIR TOO COOL FOR SCHOOL ART CLASS^{®}** (i.e.: Mintel product No.4891985) in the form of a water-in-oil emulsion, the ingredient list of which is:
   WATER, PHENYL TRIMETHICONE, ALCOHOL DENAT., DIMETHICONE, ISODODECANE, ETHYLHEXYL METHOXYCINNAMATE, SILICA, BUTYLENE GLYCOL, PEG-10 DIMETHICONE, TITANIUM DIOXIDE, METHYL METHACRYLATE CROSSPOLYMER, POLYMETHYL SILSESQUIOXANE, MICA, SODIUM CHLORIDE, DIMETHICONE/PEG-10/15 CROSSPOLYMER, ISOHEXADECANE, SQUALANE, POTASSIUM SORBATE, SODIUM DEHYDROACETATE, DISODIUM STEAROYL GLUTAMATE, TOCOPHERYL ACETATE, FRAGRANCE, ISOPROPYL TITANIUM TRIISOSTEARATE, MALTODEXTRIN, DRUMSTICK SEED EXTRACT, XYLITYL GLUCOSIDE, ALUMINIUM HYDROXIDE, ANHYDROXYLITOL, XYLITOL, DIPROPYLENE GLYCOL, CITRONELLOL, BENZYL BENZOATE, BUTYLPHENYL METHYLPROPIONAL, LINALOOL, SODIUM CITRATE, ALPHA-ISOMETHYL IONONE, COUMARIN, TOCOPHEROL, TITANIUM DIOXIDE, YELLOW IRON OXIDE, RED IRON OXIDE, BLACK IRON OXIDE.

A sensory test was performed on a panel of 14 women with normal skin. Each evaluating person, skilled in working with foundations, was trained by a reference foundations expert regarding the score attributed to reference products for the transfer-resistance criterion. The participants are foundation users who show great interest in cosmetic products. Their memory, rigour, mathematical logic, concentration and motivational capacities enabled them to meet the conditions for gaining access to the status of participants on conclusion of a training course. The protocol used is an *in vivo* protocol that is as close as possible to the gestures used by consumers, thus representing a natural gesture for personally applying a foundation formulation. The transfer-resistance criterion was evaluated on a scoring scale from 0 to 5. The higher the value, the greater the transfer.

The results obtained are indicated in the table below with the mean of the scores obtained:

| **Composition** | **Example 1 (invention)** | **WATER BLEND FACE & BODY FOUNDATION^{®} (reference)** | **LIQUID AIR TOO COOL FOR SCHOOL ART CLASS^{®} (reference)** |
|---|---|---|---|
| Transfer-resistance effect | 2.7 | 3.9 | 4.2 |

Composition 1 according to the invention in the form of an aqueous coloured particle dispersion produced a significantly better transfer-resistance effect than the reference aqueous formulations on the market of foundations in water-in-oil emulsion form.

## Claims

1. Composition in the form of an aqueous particle dispersion, especially comprising a physiologically acceptable medium, especially for coating keratin materials, more particularly for making up and/or caring for keratin materials such as the skin, containing:
a) an aqueous phase; and
b) at least one matt-effect filler **chosen from boron nitride, polymethylsilsesquioxane powders, and mixtures thereof;**
**c) solid particles formed from a film-forming polymer suspended in the aqueous phase which are an aqueous dispersion of aminomethylpropanol salt of a copolymer of allyl methacrylate and of one or more monomers chosen from acrylic acid, methacrylic acid or an ester thereof of INCI name: AMP-acrylates/allyl methacrylate copolymer; and**
d) at least one ionic polymeric dispersant **chosen from homopolymers of the sodium salt of methacrylic acid;**
e) at least one thickener **chosen from:**
- **copolymers of at least two monomers chosen from acrylic acid, methacrylic acid or an ester;**
- **polyacrylic acid/C₁₀-C₃₀ alkyl acrylate crosslinked copolymers;**
- **an acrylamidomethylpropanesulfonic acid polymer partially neutralized with ammonia and crosslinked, and mixtures thereof, and**
f) at least one particulate dyestuff **chosen from titanium dioxides, iron oxides, and mixtures thereof.**

2. Composition according to Claim 1, in which the matt-effect filler(s) are present in concentrations ranging from 0.1% to 5% by weight, more preferentially from 1% to 3% by weight, relative to the total weight of the composition.

3. Composition according to any one of the preceding claims, in which the film-forming polymer(s) are present in a solids content ranging from 0.1% to 5% by weight, more preferentially ranging from 0.1% to 3% by weight, even more preferentially ranging from 0.1% to 2% by weight and more particularly from 0.1% to 1.5% by weight relative to the total weight of the composition.

4. Composition according to any one of the preceding claims, including a total content of ionic polymeric dispersant(s) of greater than or equal to 0.05% by weight and less than or equal to 3% by weight, in particular ranging from 0.1% to 2% by weight, more particularly ranging from 0.1% to 1.5% by weight and even more particularly ranging from 0.1% to 1% by weight, relative to the total weight of the composition.

5. Composition according to any one of the preceding claims, in which the thickener(s) are present in a proportion of from 0.05% to 3% by weight of solids relative to the total weight of the aqueous phase, especially from 0.1% to 2% by weight relative to the total weight of the composition.

6. Composition according to any one of the preceding claims, in which the particulate dyestuff(s) are present in contents ranging from 5% to 40% by weight, more particularly from 10% to 30% by weight and better still from 15% to 25% by weight, relative to the total weight of the composition.

7. Composition according to any one of the preceding claims, in which the aqueous phase is present in a content ranging from 70% to 95% by weight relative to the total composition, preferably in a content ranging from 80% to 90% by weight relative to the total weight of the composition.

8. Composition according to any one of the preceding claims, in which water is present in a content ranging from 40% to 80% by weight relative to the total composition, preferably in a content ranging from 60% to 70% by weight relative to the total weight of the composition.

9. Composition according to any one of the preceding claims, containing at least one additive chosen from polyols, monoalcohols, and mixtures thereof.

10. Composition according to any one of the preceding claims, which is in the form of a foundation.

11. Composition according to any one of the preceding claims, which is in the form of an eyeshadow or a face powder.

12. Process for coating keratin materials, more particularly for making up and/or caring for keratin materials, such as the skin, **characterized in that** it comprises the application to the keratin materials of a composition as defined according to any one of the preceding claims

## Patentansprüche

1. Zusammensetzung in Form einer wässrigen Teilchendispersion, insbesondere umfassend ein physiologisch unbedenkliches Medium, insbesondere zum Beschichten von Keratinmaterialien, spezieller zum Schminken und/oder Pflegen von Keratinmaterialien wie der Haut, enthaltend:
a) eine wässrige Phase und
b) mindestens einen Matteffekt-Füllstoff, ausgewählt aus Bornitrid, Polymethylsilsesquioxan-Pulvern und Mischungen davon;
c) feste Teilchen aus einem filmbildenden Polymer, die in der wässrigen Phase suspendiert sind, bei denen es sich um eine wässrige Dispersion des Aminomethylpropanolsalzes eines Copolymers von Allylmethacrylat und einem oder mehreren Monomeren, die aus Acrylsäure, Methacrylsäure oder einen Ester davon ausgewählt sind, mit dem INCI-Namen AMP-Acrylates/Allyl Methacrylate Copolymer handelt; und
d) mindestens ein ionisches polymeres Dispergiermittel, ausgewählt aus Homopolymeren des Natriumsalzes von Methacrylsäure;
e) mindestens einen Verdicker, ausgewählt aus:
- Copolymere von mindestens zwei Monomeren, ausgewählt aus Acrylsäure, Methacrylsäure oder einem Ester;
- vernetzten Polyacrylsäure-C₁₀-C₃₀-alkylacrylat-Copolymeren;
- einem teilweise mit Ammoniak neutralisiert und vernetzten Acrylamidomethylpropansulfonsäure-Polymer und Mischungen davon; und
f) mindestens einem teilchenförmigem Farbstoff, ausgewählt aus Titandioxiden, Eisenoxiden und Mischungen davon.

2. Zusammensetzung nach Anspruch 1, wobei der Matteffekt-Füllstoff bzw. die Matteffekt-Füllstoffe in Konzentrationen im Bereich von 0,1 bis 5 Gew.-%, weiter bevorzugt von 1 bis 3 Gew.-%, bezogen auf das Gesamtgewicht der Zusammensetzung, vorliegt bzw. vorliegen.

3. Zusammensetzung nach einem der vorhergehenden Ansprüche, wobei das filmbildende Polymer bzw. die filmbildenden Polymere in einem Feststoffgehalt im Bereich von 0,1 bis 5 Gew.-%, weiter bevorzugt im Bereich von 0,1 bis 3 Gew.-%, noch weiter bevorzugt im Bereich von 0,1 bis 2 Gew.-% und spezieller von 0,1 bis 1,5 Gew.-%, bezogen auf das Gesamtgewicht der Zusammensetzung, vorliegt bzw. vorliegen.

4. Zusammensetzung nach einem der vorhergehenden Ansprüche, enthaltend einen Gesamtgehalt an ionischem polymerem Dispergiermittel bzw. ionischen polymeren Dispergiermitteln größer oder gleich 0,05 Gew.-% und kleiner oder gleich 3 Gew.-%, insbesondere im Bereich von 0,1 bis 2 Gew.-%, spezieller im Bereich von 0,1 bis 1,5 Gew.-% und noch spezieller im Bereich von 0,1 bis 1 Gew.-%, bezogen auf das Gesamtgewicht der Zusammensetzung.

5. Zusammensetzung nach einem der vorhergehenden Ansprüche, wobei der Verdicker bzw. die Verdicker in einem Anteil von 0,05 bis 3 Gew.-% Feststoffen, bezogen auf das Gesamtgewicht der wässrigen Phase, insbesondere von 0,1 bis 2 Gew.-%, bezogen auf das Gesamtgewicht der Zusammensetzung, vorliegen.

6. Zusammensetzung nach einem der vorhergehenden Ansprüche, wobei der teilchenförmige Farbstoff bzw. die teilchenförmigen Farbstoffe in Gehalten im Bereich von 5 bis 40 Gew.-%, weiter bevorzugt von 10 bis 30 Gew.-% und noch besser von 15 bis 25 Gew.-%, bezogen auf das Gesamtgewicht der Zusammensetzung, vorliegt bzw. vorliegen.

7. Zusammensetzung nach einem der vorhergehenden Ansprüche, wobei die wässrige Phase in einem Gehalt im Bereich von 70 bis 95 Gew.-%, bezogen auf die gesamte Zusammensetzung, vorzugsweise in einem Gehalt im Bereich von 80 bis 90 Gew.-%, bezogen auf das Gesamtgewicht der Zusammensetzung, vorliegt.

8. Zusammensetzung nach einem der vorhergehenden Ansprüche, wobei Wasser in einem Gehalt im Bereich von 40 bis 80 Gew.-%, bezogen auf die gesamte Zusammensetzung, vorzugsweise in einem Gehalt im Bereich von 60 bis 70 Gew.-%, bezogen auf das Gesamtgewicht der Zusammensetzung, vorliegt.

9. Zusammensetzung nach einem der vorhergehenden Ansprüche, enthaltend mindestens ein Additiv, ausgewählt aus Polyolen, Monoalkoholen und Mischungen davon.

10. Zusammensetzung nach einem der vorhergehenden Ansprüche, die in Form einer Make-up-Grundierung vorliegt.

11. Zusammensetzung nach einem der vorhergehenden Ansprüche, die in Form eines Lidschattens oder eines Gesichtspuders vorliegt.

12. Verfahren zum Beschichten von Keratinmaterialien, spezieller zum Schminken und/oder Pflegen von Keratinmaterialien wie der Haut, **dadurch gekennzeichnet, dass** es das Aufbringen einer Zusammensetzung gemäß einem der vorhergehenden Ansprüche auf die Keratinmaterialien umfasst.

## Revendications

1. Composition sous la forme d'une dispersion aqueuse de particules, comprenant notamment un milieu physiologiquement acceptable, notamment pour le revêtement de matières kératiniques, plus particulièrement pour le maquillage et/ou l'entretien pour des matières kératiniques telles que la peau, contenant :
a) une phase aqueuse ; et
b) au moins une charge à effet matifiant choisie parmi le nitrure de bore, des poudres de polyméthylsilsesquioxane, et des mélanges correspondants ;
c) des particules solides formées d'un polymère filmogène mis en suspension dans la phase aqueuse qui sont une dispersion aqueuse d'un sel d'aminométhylpropanol d'un copolymère de méthacrylate d'allyle et d'un ou plusieurs monomères choisis parmi l'acide acrylique, l'acide méthacrylique ou un ester correspondant de nom INCI : AMP/acrylates/allyl méthacrylate copolymer ; et
d) au moins un dispersant polymérique ionique choisi parmi des homopolymères du sel de sodium d'acide méthacrylique ;
e) au moins un épaississant choisi parmi :
- des copolymères d'au moins deux monomères choisis parmi l'acide acrylique, l'acide méthacrylique ou un ester ;
- des copolymères réticulés de poly(acide acrylique)/acrylates d'alkyle en C₁₀₋₃₀ ;
- un polymère d'acide acrylamidométhylpropanesulfonique partiellement neutralisé par de l'ammoniac et réticulé, et des mélanges correspondants, et
f) au moins une matière colorante particulaire choisie parmi des dioxydes de titane, des oxydes de fer, et des mélanges correspondants.

2. Composition selon la revendication 1, dans laquelle la ou les charges à effet matifiant sont présentes en des concentrations allant de 0,1 % à 5 % en poids, plus préférentiellement de 1 % à 3 % en poids, par rapport au poids total de la composition.

3. Composition selon l'une quelconque des revendications précédentes, dans laquelle le ou les polymères filmogènes sont présents en une teneur en solides dans la plage de 0,1 % à 5 % en poids, plus préférentiellement dans la plage de 0,1 % à 3 % en poids, encore plus préférentiellement dans la plage de 0,1 % à 2 % en poids, et plus particulièrement de 0,1 % à 1,5 % en poids par rapport au poids total de la composition.

4. Composition selon l'une quelconque des revendications précédentes, comprenant une teneur totale en dispersant (s) polymérique(s) ionique(s) supérieure ou égale à 0,05 % en poids et inférieure ou égale à 3 % en poids, en particulier dans la plage de 0,1 à 2 % en poids, plus particulièrement dans la plage de 0,1 % à 1,5 % en poids, et plus particulièrement encore dans la plage de 0,1 % à 1% en poids, par rapport au poids total de la composition.

5. Composition selon l'une quelconque des revendications précédentes, dans laquelle l'épaississant ou les épaississants sont présents en une proportion allant de 0,05 % à 3 % en poids de solides par rapport au poids total de la phase aqueuse, notamment de 0,1 % à 2 % en poids par rapport au poids total de la composition.

6. Composition selon l'une quelconque des revendications précédentes, dans laquelle la ou les matières colorantes particulaires sont présentes en des teneurs dans la plage de 5 % à 40 % en poids, plus particulièrement de 10 % à 30 % en poids, et encore mieux de 15 % à 25 % en poids, par rapport au poids total de la composition.

7. Composition selon l'une quelconque des revendications précédentes, dans laquelle la phase aqueuse est présente en une teneur dans la plage de 70 % à 95 % en poids par rapport à la composition totale, préférablement en une teneur dans la plage de 80 % à 90 % en poids par rapport au poids total de la composition.

8. Composition selon l'une quelconque des revendications précédentes, dans laquelle l'eau est présente en une teneur dans la plage de 40 % à 80 % en poids par rapport à la composition totale, préférablement en une teneur dans la plage de 60 % à 70 % en poids par rapport au poids total de la composition.

9. Composition selon l'une quelconque des revendications précédentes, contenant au moins un additif choisi parmi des polyols, des monoalcools et des mélanges correspondants.

10. Composition selon l'une quelconque des revendications précédentes, qui est sous la forme d'un fond de teint.

11. Composition selon l'une quelconque des revendications précédentes, qui est sous la forme d'un fard à paupières ou d'une poudre pour le visage.

12. Procédé pour le revêtement de matières kératiniques, plus particulièrement pour le maquillage et/ou l'entretien pour des matières kératiniques, telles que la peau, **caractérisé en ce qu'**il comprend l'application sur les matières kératiniques d'une composition telle que définie dans l'une quelconque des revendications précédentes.
